# EUROPÄISCHE PATENTSCHRIFT

(11) **EP 1 850 836 B1**
(45) Veröffentlichungstag und Bekanntmachung des Hinweises auf die Patenterteilung: **02.04.2014**
(21) Anmeldenummer: 06706919.5
(22) Anmeldetag: 14.02.2006
(51) Int. Cl.: A61K 9/70

(54) **MEDIKAMENTÖSE KOMBINATIONSBEHANDLUNG**
TREATMENT WITH A MEDICAMENT COMBINATION
TRAITEMENT MEDICAMENTEUX COMBINE

(30) Priorität: 21.02.2005 DE 102005007859
(43) Veröffentlichungstag der Anmeldung: 07.11.2007
(73) Patentinhaber: LTS LOHMANN Therapie-Systeme AG, 56626 Andernach (DE)
(72) Erfinder: HILLE, Thomas, 56567 Neuwied (DE); WESSLING, Werner, 56579 Rengsdorf (DE)
(74) Vertreter: Michalski Hüttermann & Partner Patentanwälte
(86) Internationale Anmeldenummer: PCT/EP2006/001312
(87) Internationale Veröffentlichungsnummer: WO 2006/087160

(56) Entgegenhaltungen:
- WO-A-2004/004681
- DE-A1- 10 256 775
- DE-A1- 19 652 188
- US-A- 5 240 711
- US-A- 5 362 496

## Beschreibung

Die Erfindung betrifft Verfahren zur Verabreichung von pharmazeutischen Wirkstoffen an Patienten, die auf die Verabreichung dieser Wirkstoffe angewiesen sind, zum Zwecke einer medikamentösen Behandlung, insbesondere zur Durchführung einer Dauertherapie. Die Erfindung betrifft ferner Kombinationen von Arzneimitteln, die für diese Verfahren geeignet sind, sowie die Verwendung von pharmazeutischen Wirkstoffen bei den genannten therapeutischen Verfahren.

Insbesondere bezieht sich die Erfindung auf eine Kombinationsbehandlung mittels transdermaler therapeutischer Systeme und gleichzeitiger Verabreichung eines oder mehrerer Wafer zur transmucosalen Wirkstoffabgabe in der Mundhöhle.

Die erfindungsgemäße Kombinationsbehandlung läßt sich besonders vorteilhaft zur therapeutischen Behandlung von Patienten einsetzen, die eine über einen längeren Zeitraum andauernde Dauertherapie oder Basisversorgung mit einem pharmazeutischen Wirkstoff benötigen, und bei denen darüber hinaus zu Beginn oder während der Dauertherapie die Notwendigkeit besteht, einen schnellen oder beschleunigten Eintritt der Arzneimittelwirkung herbeizuführen, oder/und einen während der Dauertherapie vorübergehend und unerwartet auftretenden erhöhten Wirkstoff-Bedarf zu behandeln.

Dieses Problem ist insbesondere bei der Behandlung von schweren oder chronischen Schmerzen (z. B. bei der Schmerztherapie von Tumorpatienten) von Bedeutung. Hierbei wird zur Erzielung einer anhaltenden Schmerzlinderung eine Dauer- oder Basistherapietherapie mit Analgetika (z. B. Opioide) durchgeführt, wobei die verabreichte Dosis an die vom Patienten wahrgenommene Intensität des Schmerzes angepaßt wird. Ziel ist dabei eine ausreichende Schmerzreduktion bei Vermeidung einer Überdosierung.

Allerdings können während einer solchen Dauer- oder Basistherapie mit einer individuell eingestellten Wirkstoffdosis sogenannte Durchbruchsschmerzen auftreten. Unter "Durch-bruchsschmerzen" versteht man Schmerzen, die bei oder trotz einer ständigen Schmerzmitteleinnahme nach Zeitplan (Dauermedikation) zwischendurch auftreten, und die von stärkerer Intensität sind als der mit der Dauertherapie behandelte Schmerz. Häufig haben Durchbruchschmerzen einen identifizierbaren und damit vermeidbaren Auslöser, so z.B. willkürliche Bewegungen, bestimmte Körperhaltungen, Berührung oder bei Dauerschmerzen im Magen/Darmbereich auch bestimmte Speisen. Zur Behandlung dieser Durchbruchschmerzen muß sich der Patient ein weiteres Schmerzmittel verschreiben lassen, zusätzlich zu dem im Rahmen der Basistherapie verabreichten Schmerzmittel, bei dem es sich im allgemeinen um ein Retard-Präparat handelt.

Die transdermale Applikation von Arzneistoffen (pharmazeutischen Wirkstoffen), insbesondere mittels transdermaler therapeutischer Systeme (TTS), weist eine Reihe von allgemein bekannten Vorteilen auf, z. B. eine kontrollierte und lang anhaltende Wirkstoff-Abgabe, sowie die Umgehung des "First-Pass"-Effekts. Diesen Vorteilen steht aber oft als Nachteil gegenüber, dass die Aufnahme von Arzneistoffen über die Haut in qualitativer wie auch quantitativer Hinsicht limitiert ist, und dass die Resorption des Wirkstoffs durch die Haut nach der Applikation eines TTS auf die Haut nur mit großer zeitlicher Verzögerung einsetzt.

Dem Fachmann ist bekannt, dass die Haut kein Resorptionsorgan ist, sondern vielmehr die Aufgabe hat, das Eindringen von Fremdkörpern, also auch von Arzneistoffen, zu verhindern. Somit ist diese Eigenschaft der Haut für die erwähnte zeitliche Verzögerung des Wirkungseintritts verantwortlich. Für dieses Phänomen wurde der Begriff "lag time" geprägt. Hierunter versteht man die Zeit, die zwischen der ersten Applikation eines transdermal applizierbaren Arzneimittels, z. B. eines TTS, und dem ersten Auftreten einer messbaren Plasmakonzentration bzw. dem ersten Auftreten der erwarteten physiologischen Wirkung des Pharmakons liegt.

Diese "lag time" ist dann besonders kritisch, wenn ein Arzneistoff nicht nur zum Zwecke einer Basis- oder Dauertherapie, d. h. über einen längeren Zeitraum, appliziert werden soll, sondern wenn zusätzlich noch gefordert wird, dass die Wirkung dieses Arzneistoffs möglichst unmittelbar nach der ersten Applikation des Arzneimittels eintreten soll, z. B. bei der Gabe von zentral wirksamen Schmerzmitteln. Zwar lässt sich die nachteilige "lag time" dadurch umgehen oder verkürzen, dass man bei der erstmaligen Applikation eines TTS oder bei durchbrechenden Schmerzen zusätzlich ein Arzneimittel mit schneller Wirkstofffreisetzung verabreicht, z. B. eine intravenöse Injektion. Eine solche kombinierte Applikation ist jedoch nicht unproblematisch, da eine intravenöse Injektion zwingend von einem Arzt vorgenommen werden muss. Die Gabe von Tabletten bei gleichzeitiger Applikation von TTS ist auch nicht hilfreich, da auch die gastrointestinale Absorption von Opiaten nur verzögert einsetzt.

Die Gabe von Tabletten hat außerdem zur Folge, dass der Wirkstoff nach der Magen-/Darmpassage die Leber passiert und in der Leber verstoffwechselt, d. h. unwirksam gemacht wird. Dem Fachmann ist dieses Phänomen als sogenannter "first pass effect" bekannt. Besonders bei den Opiaten mit freier Hydroxylgruppe an einem aromatischen Ring des Morphinan-Grundgerüsts (z. B. Morphin und Hydromorphon) setzt die chemische Umsetzung der Phase II, die Glucuronidierung (Konjugation mit Glucuronsäure), rasch ein.

Aufgrund der vorstehend beschriebenen Nachteile (verzögerter Wirkungseintritt) ist die transdermale Verabreichung für die Behandlung von plötzlich auftretenden Schmerzen wie z. B. Durchbruchschmerzen nicht geeignet. Daher wurde gleichzeitig mit Beginn der Entwicklung der Therapie mittels dermaler bzw. transdermaler Applikation nach Methoden gesucht, mit denen die "lag time" verkürzt und der Wirkungseintritt beschleunigt werden kann.

Eine Möglichkeit zur Beschleunigung der transdermalen Wirkstoffabsorption besteht darin, dass das auf der Haut applizierte TTS mit Ultraschall oder durch Wärmeentwicklung behandelt wird. Diese Verfahren haben den Nachteil, dass ihre praktische Durchführung schwierig ist; sie haben sich daher in der Praxis nicht durchgesetzt.

Andere Methoden zur Erhöhung der Resorptionsrate von Arzneistoffen bei transdermaler Verabreichung beruhen darauf, dass das Stratum Corneum der Haut durch Laserbehandlung oder durch wiederholtes Aufkleben und Abreißen von Klebestreifen (das sogenannte "Stripping") entfernt oder partiell geschädigt wird. Diese beiden Behandlungsmethoden verkürzen zwar ebenfalls die "lag time", bei diesen Verfahren ist jedoch nachteilig, dass nicht nur das erwünschte Penetrieren des Arzneistoffs, sondern auch ein unerwünschtes Eindringen anderer Bestandteile des Arzneimittels sowie von Mikroorganismen, wie Bakterien, Pilzsporen etc. in den menschlichen Körper erleichtert wird. Das Verfahren hat ferner den Nachteil, dass das TTS abgenommen werden muss, um die Haut zu "strippen". Unter Fachleuten ist aber bekannt, dass das Abziehen eines TTS zum Verlust der Klebkraft führt, da die oberste Hautschicht, die mit dem Kleber des TTS in Kontakt steht, ebenfalls mit entfernt wird. WO 2004/004681 beschreibt die Kombination eines TTS mit einem Aerosol. Das TTS sorgt für die Deckung des Basisbedarfs und mit dem Aerosol werden Bolusgaben verabreicht.

Ein weiterer Weg, die dermale Resorptionsgeschwindigkeit zu verbessern, besteht in der Anwendung von elektrischem Strom. Dieses unter dem Begriff Iontophorese bekannte Verfahren kann, wie dem medizinischen Fachmann bekannt ist, nicht schmerzfrei angewandt werden. Dasselbe gilt für das sogenannte Stachelpflaster; diese Form eines dermalen Arzneimittels wird mit Kanülen, die die Haut durchdringen, am Körper fixiert. Die Wirkstoffabgabe erfolgt durch die Kanülen, die gleichzeitig als Fixierhilfe dienen. Es ist offensichtlich, dass hierbei nicht mehr von dermaler bzw. transdermaler Applikation im klassischen Sinne des Wortes die Rede sein kann, sondern von subkutaner Injektion eines Arzneistoffes, mit all ihren bekannten Nachteilen (Notwendigkeit von sterilen Kanülen, keine protrahierte Abgabe etc.).

Die Aufgabe der Erfindung ist daher die Bereitstellung eines Therapieverfahrens, welches die Verabreichung eines Arzneistoffs an einen einem Patienten zur Durchführung einer Basis- oder Dauertherapie , d. h. über einen längeren Zeitraum verabreicht, ermöglicht, und welches die vorstehend erwähnten Nachteile (insbesondere "lag time" und "first-pass"-Effekt") reduziert oder vermeidet. Insbesondere bestand die Aufgabe darin, ein medikamentöses Behandlungsverfahren bereitzustellen, das die Einleitung und Aufrechterhaltung einer Basis- oder Dauertherapie ermöglicht, und wobei die therapeutische Wirkung möglichst unmittelbar nach der ersten Verabreichung eintreten soll. Mit anderen Worten: Die "lag time" soll minimiert werden.

Des weiteren bestand die Aufgabe darin, Behandlungsverfahren aufzuzeigen, mit denen in Zeiträumen, die während einer Dauertherapie auftreten und die durch einen erhöhten Arzneistoffbedarf des jeweiligen Patienten gekennzeichnet sind, die Verabreichung zumindest einer zusätzlichen Arzneistoffdosis mit möglichst geringer "lag time" ermöglicht wird.

Ferner bestand die Aufgabe der Erfindung darin, Mittel bereitzustellen, die für die Durchführung der genannten Verfahren geeignet sind.

Diese Aufgaben werden erfindungsgemäß durch die in Anspruch 1 beschriebene Kombination von Arzneimitteln gelöst, sowie durch die in den weiteren Ansprüchen definierten Erzeugnisse und therapeutischen Verwendungen.

Demnach betrifft die vorliegende Erfindung ein Verfahren zur Verabreichung zumindest eines pharmazeutischen Wirkstoffs an einen Patienten, der auf die Verabreichung dieses Wirkstoffs bzw. dieser Wirkstoffe angewiesen ist. Insbesondere handelt es sich dabei um ein Verfahren zur Durchführung einer Dauertherapie. Das erfindungsgemäße Behandlungsverfahren umfaßt:
a) das Applizieren mindestens eines transdermalen therapeutischen Systems (TTS) enthaltend einen ersten pharmazeutischen Wirkstoff, zur transdermalen Verabreichung dieses Wirkstoffs während einer vorherbestimmbaren Zeitdauer, und
b) das Applizieren mindestens eines Wafers zu Beginn oder während der Zeitdauer der transdermalen Verabreichung, wobei der Wafer denselben Wirkstoff oder einen zweiten oder weiteren Wirkstoff, der für dieselbe Indikation geeignet ist wie der erste Wirkstoff, enthält.

Durch die Applikation eines TTS wird die für die Einleitung oder/und Aufrechterhaltung einer Dauertherapie erforderliche Wirkstoff-Dosis bereitgestellt und mit verzögerter, kontrollierter Freisetzung an die Haut des Patienten abgegeben und systemisch verfügbar gemacht. Die Zeitdauer der transdermalen Verabreichung hängt im wesentlichen von der Gesamtmenge des in dem jeweiligen TTS enthaltenen Wirkstoffs, der Art des enthaltenen Wirkstoffs, der Abgabefläche des TTS und der Freisetzungsgeschwindigkeit ab. Im allgemeinen liegt die Freisetzungsdauer eines auf die Haut eines Patienten applizierten TTS im Bereich von ca. 6 bis 72 h, insbesondere 12 bis 24 oder 48 h. Nach dieser vorherbestimmbaren Zeit wird das verbrauchte TTS abgenommen und, falls erforderlich, durch ein neues TTS ersetzt. Die Gesamtdauer einer Dauertherapie kann einen oder mehrere Tage betragen, oder sie kann sich über einen unbestimmten Zeitraum erstrecken, solange die Indikation fortbesteht.

Bei dem in (b) genannten Wafer handelt es sich um eine oblatenförmige, dünne und biegsame Darreichungsform, die vorzugsweise oral appliziert wird und den/die enthaltenen Wirkstoff(e) in der Mundhöhle freisetzt, wobei die Wirkstoffresorption überwiegend über die Mundschleimhaut (d. h. transmucosal) erfolgt. Aufgrund der geringen Dicke dieser Wafer (vorzugsweise 0,05 bis 1 mm, insbesondere 0,1 bis 0,5 mm) und der dadurch bedingten kurzen Diffusionswege beginnt die Wirkstofffreisetzung unmittelbar nach dem Einbringen des Wafers in die Mundhöhle. Durch die transmucosale Resorption wird innerhalb weniger Minuten (ca. 5 bis 15 min) nach oraler Verabreichung eines Wafers ein therapeutisch wirksamer Plasmaspiegel erreicht. Dadurch wird ein rascher Wirkungseintritt ermöglicht. Vorzugsweise werden Wafer verwendet, die mucoadhäsiv oder/und in wässrigen Medien (Körperflüssigkeiten, insbesondere Speichel) zerfallsfähig sind.

Der Wafer wird gemäß (b) zu Beginn oder während der Zeitdauer der transdermalen Verabreichung appliziert. Dies bedeutet, dass der Wafer entweder zu Beginn der transdermalen Verabreichung appliziert wird (d. h. zum Zeitpunkt, in dem ein TTS auf die Haut appliziert wird), oder dass der Wafer zu einem späteren Zeitpunkt, wenn sich das TTS schon auf der Haut des Patienten befindet und die transdermale Wirkstoffabgabe bereits begonnen hat, an den Patienten verabreicht wird.

Im einfachsten Fall enthält ein Wafer, der zu Beginn oder während der Zeitdauer der transdermalen Verabreichung appliziert wird, denselben Wirkstoff oder dieselbe Wirkstoffkombination wie das TTS, mit dem die transdermale Verabreichung bewirkt wird. Alternativ, oder auch zusätzlich, kann ein derartiger Wafer einen zweiten oder weiteren Wirkstoff enthalten, der nicht mit dem im TTS enthaltenen (ersten) Wirkstoff identisch ist, der aber für dieselbe Indikation geeignet ist wie dieser erste Wirkstoff. Hierbei kann es sich um ein pharmakologisch gleichwirkendes Arzneimittel handeln, im Fall von Analgetika z. B. um ein anderes Opioid. Falls das TTS eine Kombination von zwei oder mehreren Wirkstoffen enthält, kann der gemäß (b) zusätzlich verabreichte Wafer wahlweise nur einen der Wirkstoffe der Wirkstoffkombination enthalten.

Durch die erfindungsgemäße Kombination einer transdermalen Verabreichung mit der Verabreichung eines oder mehrerer Wafer wird die Durchführung einer Dauertherapie ermöglicht, die sich durch einen raschen Wirkungseintritt auszeichnet, und die eine rasche Dosis-Anpassung ermöglicht, wenn während des Zeitraums der Dauertherapie Krankheitsphasen auftreten, in denen der Wirkstoffbedarf vorübergehend erhöht ist, insbesondere zur Behandlung von Durchbruchschmerzen in der Langzeit-Schmerztherapie. Somit eigenen sich die erfindungsgemäßen Verfahren vorzugsweise zur Behandlung solcher Patienten, bei denen zu Beginn einer Dauertherapie, oder während des Zeitraums einer Dauertherapie, ein schnelles oder beschleunigtes Eintreten der therapeutischen Wirkung erforderlich ist, oder zur Behandlung von Patienten, bei denen während der genannten Dauertherapie oder Basisversorgung ein zeitweise erhöhter Wirkstoffbedarf auftritt.

Nach einer bevorzugten Ausführungsform der Erfindung ist deshalb vorgesehen, dass in einem ersten Schritt des Behandlungsverfahrens ein TTS, welches einen ersten Wirkstoff enthält, appliziert wird und zusätzlich ein Wafer appliziert wird, der denselben Wirkstoff oder einen zweiten oder weiteren Wirkstoff enthält, der für dieselbe Indikation geeignet ist. Vorzugsweise werden das TTS und der Wafer annähernd gleichzeitig appliziert, d. h. innerhalb eines Zeitraums von weniger als 15 min, vorzugsweise weniger als 5 min). Das auf die Haut applizierte TTS verbleibt dort während der vorbestimmten Zeit (z. B. 12 bis 72 h), um die Basistherapie zu gewährleisten.
Die zusätzliche Verabreichung eines Wafers kann, wie beschrieben, vorzugsweise einmalig zu Beginn einer Basistherapie erfolgen. Falls erforderlich, können während des weiteren Verlaufs der Dauertherapie ein oder mehrere weitere Wafer verabreicht werden.

Um die Basistherapie nach Bedarf über einen längeren Zeitraum aufrecht zu erhalten, können in regelmäßigen Abständen (z. B. nach 6, 12, 24, 48 oder 72 h) weitere transdermale therapeutische Systeme mit dem genannten Wirkstoff an einen Patienten verabreicht werden, wobei das jeweils vorher applizierte, verbrauchte TTS von der Haut abgelöst wird. Auf diese Weise kann die Dauertherapie oder Basisversorgung über einen längeren Zeitraum, vorzugsweise von mindestens 24 h, fortgeführt werden. Die Dauertherapie kann sich über einen Zeitraum von mehreren Tagen, Wochen, Monaten oder Jahren erstrecken, sofern die Krankheits-Umstände dies erfordern.

Nach einer weiteren bevorzugten Ausführungsform der Erfindung weist das Behandlungsverfahren zumindest einen Schritt auf, bei dem ein transdermales therapeutisches System gemeinsam mit einem Wafer, wie oben beschrieben, verabreicht wird. Diese gemeinsame Verabreichung kann vorzugsweise zu Beginn des Behandlungsverfahrens (insbesondere zu Beginn einer Dauer- oder Basistherapie) erfolgen. Alternativ kann bei einer jeden aufeinanderfolgenden Applikation eines weiteren TTS zugleich ein Wafer appliziert werden.

Eine weitere, besonders bevorzugte Ausführungsform der Erfindung sieht vor, dass während der genannten Zeitdauer der transdermalen Verabreichung oder während der genannten Dauertherapie eine mindestens einmalige, zusätzliche Verabreichung dieses Wirkstoffs oder eines anderen Wirkstoffs, der für dieselbe Indikation geeignet ist, in Form eines Wafers erfolgt. Die durch den Wafern verabreichte Wirkstoff-Dosis ermöglicht es, einen während der genannten Zeitdauer vorübergehend auftretenden, erhöhten Wirkstoffbedarf des Patienten zu behandeln. Insbesondere wird auf diese Weise die Behandlung von Durchbruchschmerzen oder Schmerzspitzen, die während einer Langzeit-Schmerztherapie auftreten, ermöglicht. Durch die rasche systemische Verfügbarkeit des mittels Wafer transmucosal verabreichten Wirkstoffs wird eine schnelle Schmerzlinderung herbeigeführt. Die Applikation der erfindungsgemäß verwendeten Wafer kann auf einfache Weise durch den Patienten selbst erfolgen. Bei Bedarf - beispielsweise bei Durchbruchschmerzen mit besonders hoher'Intensität - können auch zwei oder mehrere Wafer gleichzeitig oder in kurzen zeitlichen Abständen verabreicht werden.

Die in einem erfindungsgemäßen Wafer enthaltene Wirkstoffmenge ("Akut-Dosis" oder "Bolus-Gabe"), welche beispielsweise bei der Einleitung einer Dauertherapie oder zu Behandlung von Durchbruchschmerzen verabreicht wird, entspricht vorzugsweise der 0,1-fachen bis 0,7-fachen, besonders bevorzugt der 0,2- bis 0,5-fachen transdermal verabreichten Tagesdosis.

Vorzugsweise werden die erfindungsgemäßen Verfahren zur Behandlung von Patienten verwendet, die an einer oder mehreren der nachfolgend genannten Krankheiten, Zuständen oder Symptomen leiden: chronische Schmerzen, Asthma, Diabetes, Herzinfarktrisiko, Nikotinentwöhnung und Parkinson'sche Krankheit. In einer besonders bevorzugten Ausführungsform des Verfahrens wird dieses zur Behandlung von Schmerzen angewendet. Bei diesen Schmerzen kann es sich um chronische und/oder akute Schmerzzustände handeln, wie sie beispielsweise bei Tumorpatienten auftreten.

Arzneistoffe, die zur Behandlung der vorstehend genannten Krankheiten, Zustände oder Symptome geeignet sind, sind dem Fachmann bekannt. Insbesondere kommen dafür Wirkstoffe in Betracht, die aus der Gruppe ausgewählt sind, die Analgetika, Broncholytika, Antidiabetika, Vasodilatoren, Entwöhnungsmittel und Parkinsonmittel umfaßt.

Allgemein können für die Zwecke der vorliegenden Erfindung alle diejenigen pharmazeutischen Wirkstoffe genutzt werden können, die transdermal applizierbar sind, da in diesen Fällen auch davon auszugehen ist, dass diese Wirkstoffe über die Mucosa des Mundes rasch resorbiert werden. Falls ein für die transdermale Verabreichung gewählter Wirkstoff nur eine unzureichende transmucosale Resorptionsrate aufweisen sollte, kann dieser - wie oben erwähnt - durch einen anderen Wirkstoff ersetzt werden, der für dieselbe Indikation geeignet ist wie der transdermal verabreichte Wirkstoff, jedoch besser transmucosal resorbiert wird.

Für die transdermale Verabreichung werden vorzugsweise solche pharmazeutische Wirkstoffe ausgewählt, die eine niedrige Hautdurchdringungsgeschwindigkeit aufweisen, so daß die angestrebte verzögerte und lang anhaltende Wirkung erreicht wird. Alternativ kann die Geschwindigkeit der WirkstoffFreisetzung aus transdermalen therapeutischen Systemen auf dem Fachmann bekannte Weise gesteuert und - falls erforderlich - reduziert werden, beispielsweise durch hierfür geeignete Hilfsstoffe oder mit Hilfe von Steuermembranen, die die Wirkstofffreisetzung verzögern.

Für die Zwecke der vorliegenden Erfindung sind vor allem solche Wirkstoffe geeignet, die hochwirksam sind, d. h. Wirkstoffe, deren Tagesdosis im Milligrammbereich liegt (z. B. 1 bis 500 mg) und deren pharmazeutisch akzeptablen Salze gut wasserlöslich sind (vorzugsweise größer 10 %, bezogen auf die Masse). Dies trifft insbesondere auf Opioide und deren Salze zu, die deshalb besonders bevorzugt verwendet werden.

Im Falle der Schmerztherapie kommen in Verbindung mit dem erfindungsgemäßen Verfahren insbesondere Analgetika, bevorzugt aus der Gruppe der Opioide, in Betracht. Mit Analgetika sind im Sinne der vorliegenden Erfindung Arzneistoffe gemeint, die in therapeutischen Dosen geeignet sind, die Schmerzempfindung zu verringern oder zu unterdrücken. Hierzu zählen insbesondere zentral angreifende stark wirksame Analgetika, die sogenannten Opioide. Zu dieser Gruppe von pharmazeutischen Wirkstoffen zählen u. a. Morphin, Heroin und weitere Derivate des Morphins; Dihydromorphinderivate wie Hydromorphon (Dihydrocodein), Oxycodon; Morphinanderivate wie Levorphanol, Buprenorphin; Analgetika der Pethidin-Gruppe, wie Pethidin, Ketobemidon, Loperamid, Diphenoxylat; Methadon und Derivate wie Levomethadon, Dextromoramit, Dextropropoxyphen; Fentanyl und seine Derivate (z. B. Alfentanil, Sufentanil, Remifentanil), Benzomorphanderivate wie Pentazocin und Phenylaminocyclohexinylderivate wie Tilidin; Tramadol. Für die Behandlung von Durchbruchschmerzen werden insbesondere schnell und kurz wirksame Opioide wie Morphin, Tramadol, Tilidin, Oxycodon, Hydromorphon, Buprenorphin, Fentanyl und Levomethadon bevorzugt.

Zum Zwecke der transdermalen Verabreichung werden Analgetika bevorzugt, die eine niedrige Hautdurchdringungsgeschwindigkeit aufweisen. Ein Beispiel hierfür ist Buprenorphin.

Aus der Gruppe der Analgetika kommen ferner beispielsweise folgende in Betracht: Metamizol, Phenazon, Propyphenazon, Flupirtin, Nefopam; Anti-Epileptika wie Carbamazepin, Gabapentin, Clonazepam; Antidepressiva wie Amitryptilin.

Die Erfindung schließt auch die Verwendung von Wirkstoffkombinationen, bestehend aus zwei oder mehreren Arzneistoffen, mit ein, insbesondere Kombinationen der vorstehend genannten Analgetika.

Es liegt auf der Hand, dass die praktische Anwendung der vorliegenden Erfindung von besonderer Bedeutung für die Gabe von Analgetika ist, da es im akuten Schmerzzustand für den Patienten unzumutbar ist, bis zum Ende der "lag time" zu warten, bis die Wirkung des Arzneimittels einsetzt. In einem solchen Fall kommt als akzeptable "lag time" ein Zeitraum von bis zu wenigen Minuten (5 bis 10 min) in Frage. Diese Bedingung wird durch die erfindungsgemäß vorgesehene Verwendung von oral applizierbaren Wafern zur transmucosalen Wirkstoff-Verabreichung erfüllt.

Die vorliegende Erfindung erstreckt sich ferner auf eine Kombination von Arzneimitteln, umfassend mindestens ein transdermales therapeutisches System (TTS) sowie mindestens einen wirkstoffhaltigen Wafer, wobei das/die TTS einen ersten pharmazeutischen Wirkstoff enthält/enthalten, und der/die Wafer denselben ersten Wirkstoff oder einen zweiten oder weiteren Wirkstoff, der für dieselbe Indikation geeignet ist wie der erste Wirkstoff, enthält/enthalten.

Allgemein bezeichnet man mit dem Begriff "Arzneimittel" Stoffe oder Stoffgemische für die Human- oder Tiermedizin, die den oder die pharmazeutischen Wirkstoff(e) sowie weitere übliche Bestandteile (inaktive Hilfsstoffe), die diesen Wirkstoff pharmazeutisch verwendbar machen, enthalten.
Die erfindungsgemäße Kombination von Arzneimitteln umfaßt Arzneimittel, die als unterschiedliche Darreichungsformen vorliegen, nämlich einerseits in Form eines TTS und andererseits in Form eines Wafers.

In der erfindungsgemäßen Arzneimittel-Kombination ist eine bestimmte Anzahl von transdermalen therapeutischen Systemen (TTS), die denselben pharmazeutischen Wirkstoff enthalten und vorzugsweise auch ansonsten eine identische Zusammensetzung aufweisen, einer bestimmten Anzahl von Wafern zugeordnet. Diese Wafer enthalten vorzugsweise denselben Wirkstoff wie das/die TTS, oder einen andersartigen Wirkstoff, der für dieselbe Indikation geeignet ist wie der in dem/den TTS enthaltene Wirkstoff. Auch im Falle der Wafer wird bevorzugt, dass alle zu einer Kombination gehörende Wafer eine im wesentlichen identische Zusammensetzung aufweisen.
Die erwähnte Zuordnung einer bestimmten Anzahl von TTS zu einer bestimmten Anzahl von Wafern kann vorzugsweise in der Weise verwirklicht werden, dass diese TTS und Wafer in einer gemeinsamen Verpackung verpackt werden und als ein "Set" oder "Kit" vorliegen.

Eine erfindungsgemäße Arzneimittel-Kombination enthält zumindest ein TTS und zumindest einen Wafer. Die Anzahl der in einer Kombination enthaltenen TTS und Wafer kann wahlweise gleich groß oder unterschiedlich sein. Insbesondere für die Anwendung bei der Schmerztherapie wird bevorzugt, dass die Wafer in einer größeren Anzahl in der Kombination enthalten sind als die transdermalen therapeutischen Systeme. Die in einer bestimmten Arzneimittel-Kombination enthaltenen Wafer weisen üblicherweise denselben Wirkstoff-Gehalt auf. Daneben kann es von Vorteil sein, wenn eine solche Kombination zwei oder mehrere Gruppen von Wafern enthält, die sich hinsichtlich ihrer Wirkstoff-Dosis unterscheiden und entsprechend gekennzeichnet sind.

Die in den transdermalen therapeutischen Systemen und Wafern der Arzneimittel-Kombination enthaltenen Wirkstoffe sind vorzugsweise aus den oben erwähnten Wirkstoffen und Wirkstoffgruppen ausgewählt. Des weiteren wird bevorzugt, dass die in der Arzneimittel-Kombination enthaltenen transdermalen therapeutischen Systeme eine systemische, transdermale Verabreichung des darin enthaltenen Wirkstoffs über einen Zeitraum von mindestens 24 h, bevorzugt mindestens 48 h, ermöglichen. Bei den in der Arzneimittel-Kombination enthaltenen Wafer handelt es sich vorzugsweise um Wafer zur oralen Verabreichung, bei denen die therapeutische Wirkung spätestens 15 min, vorzugsweise spätestens 5 min nach oraler Verabreichung einsetzt. Des weiteren wird bevorzugt, dass die Wafer mucoadhäsiv oder/und in wässrigen Medien zerfallsfähig sind.

Die Erfindung umfaßt ferner die Verwendung von pharmazeutischen Wirkstoffen, insbesondere von Wirkstoffen, die aus den oben erwähnten Wirkstoffen und Wirkstoffgruppen ausgewählt sind, zur Herstellung der oben beschriebenen, erfindungsgemäßen Arzneimittel-Kombinationen zur Behandlung von Patienten, die auf die Verabreichung eines solchen Wirkstoffs zur Erzielung einer Dauertherapie oder Basisversorgung angewiesen sind. Diese Arzneimittel-Kombinationen werden vorzugsweise bei den oben beschriebenen therapeutischen Behandlungsverfahren und für die genannten therapeutischen Zwecke verwendet.

Die erfindungsgemäß verwendeten TTS und Wafer können mit bekannten pharmazeutischen Verfahren hergestellt werden; ebenso sind die Zusammensetzungen dieser Formulierungen und die dabei verwendeten Hilfsstoffe dem Fachmann bekannt.
TTS, die für die Zwecke der vorliegenden Erfindung verwendet werden können, sind beispielsweise in DE 39 39 376 C1, DE 199 23 551 A1 und DE 198 34 005 A1 beschrieben.

Die erfindungsgemäß verwendeten TTS sind vorzugsweise schichtförmig aufgebaut, d. h. zwei-, drei- oder mehrschichtig. Insbesondere kann der schichtförmige Aufbau des TTS eine oder mehrere Schichten umfassen, die ausgewählt sind aus:
- druckempfindlichen Haftkleberschichten,
- porösen Schichten, und
- Hydrogel-Schichten.
Mindestens eine der Schichten des TTS enthält einen Wirkstoff oder eine Wirkstoffkombination, wie oben beschrieben. Vorzugsweise sind die erfindungsgemäß verwendeten TTS mit einer Haftkleberschicht ausgestattet, die der Befestigung des TTS auf der Haut des Patienten dient, und die bevorzugt wirkstoffhaltig ist.

Die wirkstoffhaltige Schicht (oder Matrix) des TTS besteht vorzugsweise aus einem haftklebenden, wasserunlöslichen Polymer, z.B. partiell veresterten Polyacrylaten, Polyisobutylen oder Silikonen, oder Mischungen solcher Polymere; zusätzlich können bekannte Hilfsstoffe beigemischt sein (z. B. Lösungsvermittler, Emulgatoren, Permeations-Enhancer, Konservierungsmittel).

Bei dem erfindungsgemäß verwendeten Wafer kann es sich, ohne die Erfindung einzuschränken, um ein flächenförmiges Gebilde zur oralen Verabreichung von Wirkstoffen handeln. Hierbei ist der Wirkstoff in einem Polymer oder Polymergemisch gelöst oder in einer Polymermatrix dispergiert. Die zu Herstellung des Wafers verwendeten Polymere sollten vorzugsweise wasserlöslich sein, damit sich der Wafer, wie beabsichtigt, in der Speichelflüssigkeit der Mundhöhle rasch, idealerweise in Sekunden (z. B. maximal 5 bis 30 s), auflösen kann.

Als Polymere für die Herstellung der Wafer kommen insbesondere Polymere aus der Gruppe der Polyethylenglykole, Stärke und Stärkederivate, Polyvinylalkohole und Polyacrylsäure (z.B. Carbopol^{®}) in Betracht, oder Polyvinylpyrrolidon (Polyvidon z.B. Kollidon^{®}). Des weiteren können die Wafer einen oder mehrere Hilfsstoffe enthalten, wie Weichmacher, Emulgatoren, Tenside, Lösungsvermittler, Füllstoffe, Zerfallshilfsmittel, Farbstoffe, Aroma- und Süßstoffe, Konservierungsmittel; derartige Hilfsstoffe sind dem Fachmann bekannt. Wafer, die für die Zwecke der vorliegenden Erfindung verwendet werden können, sowie dafür geeignete Herstellungsverfahren, sind beispielsweise in DE 102 07 304 A1 und US 6 709 671 B2 beschrieben.

### Beispiel

Die Erfindung wird durch das nachfolgende Beispiel näher erläutert.

Das Beispiel bezieht sich auf die therapeutische Behandlung eines Schmerzpatienten. Zur Dauerbehandlung bzw. für die Basistherapie wird ein (bzw. mehrere) Buprenorphin enthaltendes TTS hergestellt, wie in DE 39 39 376 C1 beschrieben (siehe folgende Tabelle). Dieses TTS wird auf die Haut eines Schmerzpatienten appliziert und dort während der vorgesehenen Applikationsdauer belassen (z. B. 24, 48 oder 72 h).

Das verwendete TTS enthält Arznei- und Hilfsstoffe gemäß nachfolgender Tabelle:

| Arznei- oder Hilfsstoff | Menge pro TTS [mg] |
|---|---|
| Buprenorphin-Base | 20 |
| Oleyloleat | 30 |
| Lävulinsäure | 20 |
| Polyacrylatkleber, vernetzt mit Aluminium | 680 |
| Polyethylenterephthalat-Gewebe als Rückschicht | 518 |
| Polyethylenterephthalat-Folie in 23 µm Dicke | 80 |
| Silikonisierte Polyethylenterephthalat-Folie als Schutzschicht | 919 |

Die Freisetzungsrate eines solchen TTS beträgt 35 µg/h (bezogen auf die Freisetzung von Buprenorphin aus jeweils einem TTS).

Gleichzeitig wird an diesen Patienten ein Wafer oral verabreicht. Dieser Wafer enthält 10 Gew.-% Buprenorphinhydrochlorid in einem Polymergemisch aus 65 Gew.-% Carbopol^{®} und 25 Gew.-% Stärke. Ein einzelner Wafer besteht im wesentlichen aus 1 mg Buprenorphinhydrochlorid, 6,5 mg Carbopol und 2,5 mg Stärke.

Die hohe Konzentration des Arzneistoffes im Wafer ermöglicht es, dass der Patient sofort bei Beginn der Langzeit-Schmerztherapie eine Schmerzlinderung erfährt, da der Wafer innerhalb von ca. 5 bis 10 s im Mund zerfällt und das freigesetzte Buprenorphin (als wasserlösliches Salz) direkt über die orale Mucosa resorbiert wird, so dass innerhalb weniger Minuten ein therapeutisch wirksamer Plasmaspiegel erreicht wird.

Falls während der Dauer der transdermalen Wirkstoffverabreichung Durchbruchschmerzen auftreten, werden möglichst frühzeitig (d. h. sobald die ersten Anzeichen der Zunahme der Schmerzintensität wahrgenommen wird) ein oder mehrere der Buprenorphin-HCl enthaltende Wafer in die Mundhöhle des Patienten appliziert. Dadurch kann der Patient aufgrund der raschen Resorption des wasserlöslichen Salzes über die Mundschleimhaut eine unmittelbare Erleichterung im akuten Schmerzzustand erfahren.

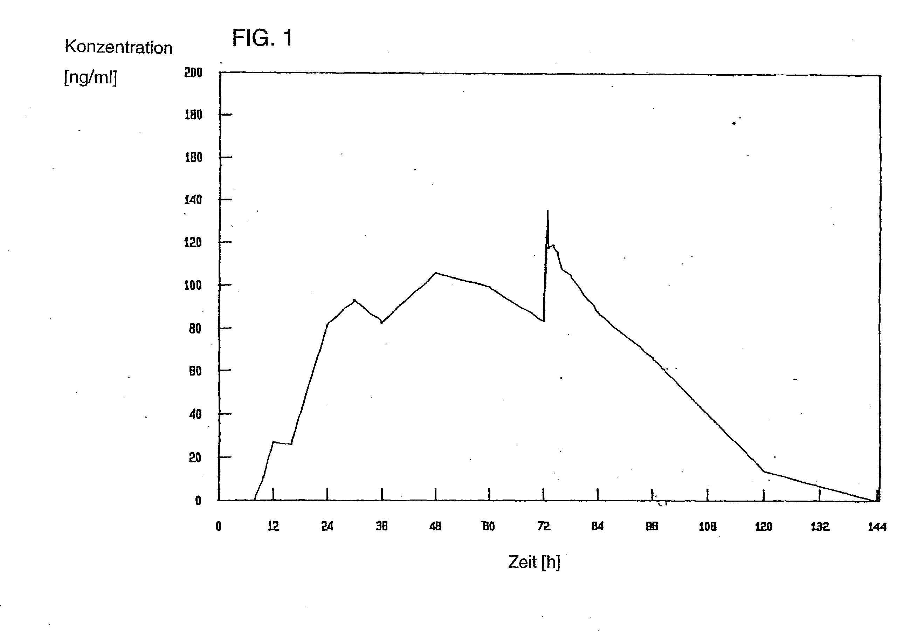

Figur 1 zeigt mittlere Plasmaspiegel, die durch das Applizieren des buprenorphinhaltigen TTS an n = 5 gesunden Freiwilligen ermittelt wurden. Es ist deutlich erkennbar, dass ein Schmerzpatient in den ersten 12 Stunden nicht ausreichend therapiert wurde; die erzielte Plasma-Konzentration liegt unter 30 ng/ml ("lag time"). Erst nach 24 Stunden ist eine Basistherapie durch das Plasmaplateau sicher erreicht (Buprenorphin-Konzentration ca. 80-100 ng/ml). Diese Plateau-Konzentration wird bis zu einer Dauer von etwa 96 h nach Applikation des TTS aufrecht erhalten.

Bei durchbrechenden Schmerzen, sowie in den ersten 12 Stunden nach Applikation des TTS, wird durch die erfindungsgemäß vorgesehene zusätzliche Gabe eines Wafers zur oralen Applikation erreicht, dass die Wirkung des Buprenorphins rasch einsetzt und der "first pass effect" vermieden wird, so dass eine schnelle und effiziente Linderung des Durchbruchschmerzes bewirkt wird.

## Patentansprüche

1. Kombination von Arzneimitteln, umfassend mindestens ein transdermales therapeutisches System (TTS) sowie mindestens einen wirkstoffhaltigen Wafer, mit dem der/die enthaltene(n) Wirkstoff(e) in der Mundhöhle freigesetzt wird/werden, wobei die Wirkstoffresorption überwiegend über die Mundschleimhaut erfolgt, wobei das/die TTS einen ersten pharmazeutischen Wirkstoff enthält/enthalten, und der/die Wafer denselben ersten Wirkstoff oder einen zweiten oder weiteren Wirkstoff, der für dieselbe Indikation geeignet ist wie der erste Wirkstoff, enthält/enthalten, und der erste Wirkstoff aus der Gruppe ausgewählt ist, die Analgetika, Broncholytika, Antidiabetika, Vasodilatoren, und Parkinsonmittel umfaßt.

2. Kombination nach Anspruch 1, **dadurch gekennzeichnet, dass** der genannte zweite oder weitere Wirkstoff aus der Gruppe ausgewählt ist, die Analgetika, Broncholytika, Antidiabetika, Vasodilatoren, und Parkinsonmittel umfaßt.

3. Kombination nach Anspruch 1 oder 2, **dadurch gekennzeichnet, dass** das/die transdermal(n) therapeutische(n) System(e) eine systemische, transdermale Verabreichung des darin enthaltenen Wirkstoffs über einen Zeitraum von mindestens 24 h, bevorzugt mindestens 48 h, ermöglichen.

4. Kombination nach einem der Ansprüche 1 bis 3, **dadurch gekennzeichnet, dass** der/die genannte(n) Wafer zur oralen Verabreichung geeignet sind, und das die therapeutische Wirkung spätestens 15 min, vorzugsweise spätestens 5 min nach oraler Verabreichung eines Wafers einsetzt.

5. Kombination nach einem der Ansprüche 1 bis 4, **dadurch gekennzeichnet, dass** der/die Wafer in wässrigen Medien zerfallsfähig oder/und mucoadhäsiv sind.

6. Verwendung zumindest eines pharmazeutischen Wirkstoffs zur Herstellung einer Kombination von Arzneimitteln, die
a) mindestens ein transdermales therapeutisches System (TTS), enthaltend einen ersten pharmazeutischen Wirkstoff aus der Gruppe, die Analgetika, Broncholytika, Antidiabetika, Vasodilatoren, und Parkinsonmittel enthält, sowie
b) mindestens einen Wafer umfaßt, der denselben Wirkstoff oder einen zweiten oder weiteren Wirkstoff, der für dieselbe Indikation geeignet ist, enthält, und mit dem der/die enthaltene(n) Wirkstoff(e) in der Mundhöhle freigesetzt wird/werden, wobei die Wirkstoffresorption überwiegend über die Mundschleimhaut erfolgt,
zur Behandlung eines Patienten, der auf die Verabreichung dieses Wirkstoffs zur Erzielung einer Dauertherapie oder Basisversorgung angewiesen ist.

7. Verwendung nach Anspruch 6, **dadurch gekennzeichnet, dass** die Kombination zur Behandlung eines Patienten geeignet ist, bei dem
- zu Beginn einer Dauertherapie, oder während des Zeitraums einer Dauertherapie, ein schnelles oder beschleunigtes Eintreten der therapeutischen Wirkung erforderlich ist; oder bei dem
- während der genannten Dauertherapie oder Basisversorgung ein zeitweise erhöhter Wirkstoffbedarf auftritt.

8. Verwendung nach Anspruch 6 oder 7, **dadurch gekennzeichnet, dass** die Dauertherapie oder Basisversorgung durch Applikation des/der genannten transdermalen therapeutischen Systems/Systems bewirkt wird, und dass der erwähnte vorübergehend erhöhte Wirkstoffbedarf durch Verabreichung eines Wafers behandelt wird.

9. Verwendung nach einem der Ansprüche 6 bis 8, **dadurch gekennzeichnet, dass** die Verabreichung eines oder mehrerer der genannten Wafer bei der Einleitung oder Aufrechterhaltung einer medikamentösen Dauertherapie vorgenommen wird, wodurch ein schneller oder beschleunigter Wirkungseintritt herbeigeführt wird.

10. Verwendung nach einem der Ansprüche 6 bis 9, **dadurch gekennzeichnet, dass** der Patient, der auf die Verabreichung des/der genannten WirkstoffsNVirkstoffe angewiesen ist, unter einem oder mehreren Krankheiten oder Symptomen leidet, die aus der Gruppe ausgewählt sind, die chronische Schmerzen, Asthma, Diabetes, Herzinfarktrisiko, Nikotinentwöhnung und Parkinson'sche Krankheit umfaßt.

11. Verwendung nach einem der Ansprüche 6 bis 10, **dadurch gekennzeichnet, dass** der genannte Wirkstoff, oder zumindest einer der genannten Wirkstoffe, aus der Gruppe ausgewählt ist, die Analgetika, Broncholytika, Antidiabetika, Vasodilatoren, Entwöhnungsmittel und Parkinsonmittel umfaßt.

## Claims

1. Combination of medicaments comprising at least one transdermal therapeutic system (TTS) and at least one active substance-containing wafer which releases the active substance(s) contained therein in the oral cavity, with the active substance absorption taking place mainly via the oral mucosa, wherein said TTS comprise(s) a first pharmaceutically active substance, and said wafer(s) comprise(s) the same first active substance or a second or further active substance suitable for the same indication as said first pharmaceutically active substance, and wherein said first active substance is selected from the group consisting of analgesics, broncholytics, antidiabetics, vasodilators and anti-Parkinson agents.

2. Combination according to claim 1, **characterized in that** the said second or further active substance is selected from the group consisting of analgesics, broncholytics, antidiabetics, vasodilators and anti-Parkinson agents.

3. Combination according to claim 1 or 2, **characterized in that** the transdermal therapeutic system(s) enable(s) a systemic, transdermal administration of the active substance contained therein over a period of at least 24 hours, preferably of at least 48 hours.

4. Combination according to any one of claims 1 to 3, **characterized in that** the said wafer(s) is/are suitable for oral administration, and that the therapeutic action starts at the latest 15 min, preferably at the latest 5 min, following oral administration of the wafer.

5. Combination according to any one of claims 1 to 4, **characterized in that** the wafer(s) is/are disintegratable in aqueous media and/or mucoadhesive.

6. Use of at least one pharmaceutically active substance for manufacturing a combination of medicaments, comprising:
(a) at least one transdermal therapeutic system (TTS) comprising a first pharmaceutically active substance from the group consisting of analgesics, broncholytics, antidiabetics, vasodilators and anti-Parkinson agents; and
(b) at least one wafer comprising the same active substance or a second or further active substance suitable for the same indication, and which releases the active substance(s) contained therein in the oral cavity, with the active substance absorption taking place mainly via the oral mucosa,
for treating a patient who depends on the administration of said active substance in order to achieve a long-term therapy or basic therapy.

7. Use according to claim 6, **characterized in that** the combination is suitable for the treatment of a patient, wherein
- a rapid or accelerated onset of therapeutic action is required at the beginning of a long-term therapy, or during long-term therapy; or wherein
- a temporarily increased active substance requirement occurs during said long-term therapy or basic therapy.

8. Use according to claim 6 or 7, **characterized in that** the long-term therapy or basic therapy is effected by application of said transdermal therapeutic system(s), and that said temporarily increased active substance requirement is treated by administration of a wafer.

9. Use according to any one of claims 6 to 8, **characterized in that** the administration of one or more of said wafers is carried out when initiating or maintaining a long-term therapy, thereby bringing about a rapid or accelerated onset of action.

10. Use according to any one of claims 6 to 9, **characterized in that** the patient who depends on the administration of said active substance(s) suffers from one or more diseases or symptoms selected from the group consisting of chronic pain, asthma, diabetes, risk of cardiac infarction, nicotine withdrawal and Parkinson's disease.

11. Use according to any one of claims 6 to 10, **characterized in that** the said active substance or at least one of said active substances is selected from the group consisting of analgesics, broncholytics, antidiabetics, vasodilators and anti-Parkinson agents.

## Revendications

1. Association de médicaments, comprenant au moins un système thérapeutique transdermique (STT) ainsi qu'au moins une gaufrette contenant un/des principe(s) actif(s) et permettant de libérer dans la cavité buccale le/les principe(s) actif(s) qu'elle contient, le(s) principe(s) actif(s) étant majoritairement absorbé(s) à travers la muqueuse buccale, le(s) STT contenant un premier principe actif pharmaceutique, et le(s) gaufrette(s) contenant le même premier principe actif ou un deuxième ou autre principe actif qui est approprié pour la même indication que le premier principe actif, et le premier principe actif étant choisi dans le groupe comprenant des analgésiques, des bronchodilatateurs, des antidiabétiques, des vasodilatateurs, et des agents visant la maladie de Parkinson.

2. Association selon la revendication 1, **caractérisée en ce que** ledit deuxième ou autre principe actif est choisi dans le groupe comprenant des analgésiques, des bronchodilatateurs, des antidiabétiques, des vasodilatateurs, et des agents visant la maladie de Parkinson.

3. Association selon la revendication 1 ou 2, **caractérisée en ce que** le(s) système(s) thérapeutique(s) transdermique(s) permet(tent) une administration systémique transdermique du principe actif qu'il(s) contien(nen)t pendant un intervalle d'au moins 24 h, préférentiellement d'au moins 48 h.

4. Association selon l'une des revendications 1 à 3, **caractérisée en ce que** ladite/lesdites gaufrette(s) sont appropriées pour une administration orale, et l'effet thérapeutique se manifeste au plus tard 15 min, préférentiellement au plus tard 5 min, après l'administration orale d'une gaufrette.

5. Association selon l'une des revendications 1 à 4, **caractérisée en ce que** la/les gaufrette(s) peuvent se désintégrer dans des milieux aqueuses ou/et sont muco-adhésives.

6. Utilisation d'au moins un principe actif pharmaceutique pour fabriquer une association de médicaments comprenant
a) au moins un système thérapeutique transdermique (STT) contenant un premier principe actif pharmaceutique appartenant au groupe des analgésiques, des bronchodilatateurs, des antidiabétiques, des vasodilatateurs et des agents visant la maladie de Parkinson, ainsi que
b) au moins une gaufrette laquelle contient le même principe actif ou un deuxième ou autre principe actif qui est approprié pour la même indication, et laquelle permet de libérer dans la cavité buccale le/les principe(s) actif(s) qu'elle contient, le(s) principe(s) actif(s) étant majoritairement absorbé(s) à travers la muqueuse buccale,
pour traiter un patient nécessitant l'administration dudit principe actif pour effectuer une thérapie permanente ou une prise en charge de base.

7. Utilisation selon la revendication 6, **caractérisée en ce que** ladite association est appropriée pour le traitement d'un patient
- nécessitant au début d'une thérapie permanente, ou pendant la mise en oeuvre d'une thérapie permanente, une manifestation rapide ou accélérée de l'effet thérapeutique ; ou
- ayant durant ladite thérapie permanente ou prise en charge de base un besoin temporairement accru en ce qui concerne le/les principe(s) actif(s).

8. Utilisation selon les revendications 6 ou 7, **caractérisée en ce que** la thérapie permanente ou la prise en charge de base est mise en oeuvre en appliquant ledit/lesdits système(s) thérapeutique(s) transdermique(s) et que ledit besoin temporairement accru en ce qui concerne le/les principe(s) actif(s) est traité par l'administration d'une gaufrette.

9. Utilisation selon l'une des revendications 6 à 8, **caractérisée en ce que** l'administration d'un ou de plusieurs desdites gaufrettes a lieu lors de la mise en place ou lors du maintien d'une thérapie médicamenteuse permanente, pour ainsi provoquer une manifestation rapide ou accélérée de l'effet.

10. Utilisation selon l'une des revendications 6 à 9, **caractérisée en ce que** le patient nécessitant l'administration dudit/desdits principe(s) actif(s) souffre d'un ou de plusieurs symptômes ou maladies choisi(e)(s) dans le groupe comprenant les douleurs chroniques, l'asthme, le diabète, le risque d'un infarctus du myocarde, le sevrage de la nicotine et la maladie de Parkinson.

11. Utilisation selon l'une des revendications 6 à 10, **caractérisée en ce que** ledit principe actif, ou au moins un desdits principes actifs, est choisi dans le groupe comprenant des analgésiques, des bronchodilatateurs, des antidiabétiques, des vasodilatateurs, des agents de sevrage et des agents visant la maladie de Parkinson.
